# EUROPEAN PATENT APPLICATION

(11) **EP 4 616 795 A1**
(43) Date of publication of application: **17.09.2025**
(21) Application number: 25162716.2
(22) Date of filing: 10.03.2025
(51) Int. Cl.: A61B 5/024, A61B 5/11, A61B 5/00

(54) **PERSONALIZED HEART RATE PREDICTION DEVICE AND METHOD**

(30) Priority: 11.03.2024 US 202418601838
(71) Applicant: Analog Devices International Unlimited Company, Limerick (IE)
(72) Inventor: HANAYLI, Suleyman, Co. Limerick (IE)
(74) Representative: Wallin, Nicholas James

(57) **Abstract**

A device for determining a personalized heart rate, including one or more heart rate sensors configured to obtain heart rate values for a user for a time period, one or more movement sensors configured to obtain movement values for the user for the time period, one or more processors configured to execute a personalized heart rate prediction model, trained on user-specific movement data and user-specific heart rate data associated with signal qualities greater than the signal quality threshold, to receive the heart rate values and the activity type or the movement values, and generate, based on the heart rate values and the activity type or the movement values, a user-specific predicted heart rate for at least a part of the time period, and a user interface configured to output the user-specific predicted heart rate as the user heart rate for at least the part of the time period.

## Description

### PRIORITY CLAIM

The present European patent application claims priority to U.S. Non-provisional Application No. 18/601,838, entitled, "PERSONALIZED HEART RATE PREDICTION DEVICE AND METHOD" filed March 11, 2024, which is hereby expressly incorporated by reference herein for all purposes.

### BACKGROUND

### Technical Field

The present disclosure relates generally to heart rate (HR) monitoring, and more particularly, to heart rate prediction using customized and personalized heart rate monitoring.

### Introduction

Wearable electronic sensors that track and provide actionable insights about a user's health and wellbeing have increased in popularity in recent years. Heart rate (HR) assessment using low power optical sensors is one of the most convenient and preferred approaches for gathering heart rate data for users. The accuracy of HR measurements depends on signal quality from these optical sensors. Unfortunately, low power optical sensors are frequently of marginal quality and can suffer from problems due to movement, poor contact, and other interference, and therefore fail to provide reliable results.

Existing technology includes modeling trends of heart rate activity, which fail to account for unique aspects of each user's movements and physiology. Existing methods are also prone to provide less reliable results and therefore fail over extended periods of time, especially during strenuous or lengthy athletic activities. Likewise, heart rate tracking by an observer using Kalman filtering fails to perform reliably, or fails to accurately measure heart rate when a monitored person's pulse rate is high during low density motion or when pulse rate is low during high density motion.

There exists a need for further improvements in opto-electrical heart rate monitoring technology. These improvements may also be applicable to other heart rate monitoring technologies and the standards that employ these technologies.

### SUMMARY

The following presents a simplified summary of one or more aspects in order to provide a basic understanding of such aspects. This summary is not an extensive overview of all contemplated aspects and is intended to neither identify key or critical elements of all aspects nor delineate the scope of any or all aspects. Its sole purpose is to present some concepts of one or more aspects in a simplified form as a prelude to the more detailed description that is presented later.

Disclosed herein are systems and methods for improving heart rate monitoring performance, even when input signal is not fully reliable or of questionable quality. Opto-electric signal quality is primarily defined by i) the quality of one or more sensors used in the monitoring system, ii) the opto-electronics and their industrial design, iii) the sensor to skin coupling, iv) the subject user's physiological condition, v) the type of physical activity occurring while being monitored, and vi) environmental conditions existing concurrent with measurement. During continuous HR assessment, photoplethysmography (PPG) signals can deteriorate to the extent that conventional frequency tracking is impossible. For example, a user may wear a smart watch during an intermittent running exercise event and wish to monitor their heart rate. While PPG signal quality may begin at a mostly adequate level, the PPG signal quality may slowly deteriorate over time or even suddenly deteriorate near the end of the event. This deterioration may cause HR monitoring signals to become diluted and impossible to track. Aspects herein provide systems and methods to accurately predict heart rate during periods of low signal quality.

In various aspects, systems can include wearable devices including sensors that report heart rate as a vital sign using PPG and ACC sensors. Kinematic information about the user can be used to predict heart rate when the quality of PPG signals degrades.

To the accomplishment of the foregoing and related ends, the one or more aspects comprise the features hereinafter fully described and particularly pointed out in the claims. The following description and the annexed drawings set forth in detail certain illustrative features of the one or more aspects. These features are indicative, however, of but a few of the various ways in which the principles of various aspects may be employed, and this description is intended to include all such aspects and their equivalents.

### BRIEF DESCRIPTION OF THE DRAWINGS

FIG. 1 is a diagram illustrating an example of a heart rate prediction architecture in accordance with an aspect of the present disclosure.
FIG. 2 is a flowchart illustrating an example of a method of monitoring and predicting a heart rate of a user in accordance with an aspect of the present disclosure.
FIG. 3 is a set of diagrams illustrating an example of a PPG signal power spectrogram, motion compensated PPG power spectrogram, heart rate monitoring algorithm with heart rate output and an electrocardiogram golden heart rate reference, and heart rate measurement confidence levels provided by a heart rate monitoring algorithm in accordance with an aspect of the present disclosure.
FIG. 4 is a set of diagrams illustrating an example of a PPG signal power spectrogram, motion compensated PPG power spectrogram, heart rate monitoring algorithm with heart rate output using an optical measurement enhanced with a heart rate prediction model and an electrocardiogram heart rate reference, and heart rate measurement confidence levels provided by a heart rate monitoring algorithm in accordance with an aspect of the present disclosure.
FIG. 5 is a set of diagrams illustrating an example of heart rate versus time and raw PPG power spectrogram in accordance with an aspect of the present disclosure.
FIG. 6 is a diagram illustrating an example of a dynamic heart rate prediction look up graph for a walking activity in accordance with an aspect of the present disclosure.
FIG. 7 is a diagram illustrating an example of a dynamic heart rate prediction look up graph for a running activity in accordance with an aspect of the present disclosure.
FIG. 8 is a diagram illustrating an example of a dynamic heart rate prediction look up graph for a biking/cycling activity in accordance with an aspect of the present disclosure.
FIG. 9 is a diagram illustrating dynamic heart rate prediction smoothing with auto-regressive filtering in accordance with an aspect of the present disclosure.
FIG. 10 is a diagram illustrating dynamic heart rate prediction smoothing with auto-regressive filtering for a biking/cycling activity in accordance with an aspect of the present disclosure.
FIG. 11 is a diagram illustrating an example of a dynamic heart rate prediction look up graph for a rowing activity in accordance with an aspect of the present disclosure.
FIG. 12 is a diagram illustrating dynamic heart rate prediction smoothing with auto-regressive filtering for a rowing activity in accordance with an aspect of the present disclosure.
FIG. 13 is a diagram illustrating dynamic heart rate prediction smoothing with auto-regressive filtering for a walking activity in accordance with an aspect of the present disclosure.
FIG. 14 is a set of diagrams illustrating a walking activity accuracy versus reporting coverage/availability in a diagram and a running activity accuracy versus reporting coverage/availability in a diagram in accordance with an aspect of the present disclosure.
FIG. 15 is a flowchart illustrating a method for determining a personalized heart rate for a user of a heart rate prediction apparatus in accordance with aspects of the present disclosure.
FIGs. 16A-16F are diagrams illustrating examples of confidence level analysis in accordance with aspects of the present disclosure.
FIGs. 17A-17D are diagrams illustrating dynamic prediction model development for a user engaged in a running activity in accordance with aspects of the present disclosure.
FIGs. 18A-18N are diagrams illustrating prior heart rate versus polar reference and MLP HR versus polar reference in accordance with aspects of the present disclosure.

### DETAILED DESCRIPTION

The detailed description set forth below in connection with the appended drawings is intended as a description of various configurations and is not intended to represent the only configurations in which the concepts described herein may be practiced. The detailed description includes specific details for the purpose of providing a thorough understanding of various concepts. However, it will be apparent to those skilled in the art that these concepts may be practiced without these specific details. In some instances, well known structures and components are shown in block diagram form in order to avoid obscuring such concepts.

Several aspects of a personalized heart rate prediction system will now be presented with reference to various apparatuses and methods. These apparatuses and methods will be described in the following detailed description and illustrated in the accompanying drawings by various blocks, components, circuits, processes, algorithms, etc. (collectively referred to as "elements"). These elements may be implemented using electronic hardware, computer software, or any combination thereof. Whether such elements are implemented as hardware or software depends upon the particular application and design constraints imposed on the overall system.

By way of example, an element, or any portion of an element, or any combination of elements may be implemented as a "processing system" that includes one or more processors. Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field programmable gate arrays (FPGAs), programmable logic devices (PLDs), state machines, gated logic, discrete hardware circuits, and other suitable hardware configured to perform the various functionality described throughout this disclosure. One or more processors in the processing system may execute software. Software shall be construed broadly to mean instructions, instruction sets, code, code segments, program code, programs, subprograms, software components, applications, software applications, software packages, routines, subroutines, objects, executables, threads of execution, procedures, functions, etc., whether referred to as software, firmware, middleware, microcode, hardware description language, or otherwise.

Accordingly, in one or more example aspects, the functions described may be implemented in hardware, software, or any combination thereof. If implemented in software, the functions may be stored on or encoded as one or more instructions or code on a computer-readable medium. Computer-readable media includes computer storage media. Storage media may be any available media that can be accessed by a computer. By way of example, and not limitation, such computer-readable media can comprise a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that can be used to store computer executable code in the form of instructions or data structures that can be accessed by a computer.

Accurate measurement of heart rate (HR) can become highly challenging, if not impossible, when the quality of photoplethysmography (PPG) signals degrade. To remedy this problem, aspects included herein allow users to monitor, store, and utilize kinematic information about their movements during different types of activities (e.g., during exercise activities such as walking, running, rowing, biking/cycling) in order to accurately predict the user's heart rate in a specialized way for each user. These aspects can include one or more models in which the inputs are motion frequency, accelerometer density, and in some cases other parameters, and outputs an expected/predicted heart rate for the specific user. To customize and specialize the model for the user, the model can be constantly or periodically trained and updated with heart rate measurements meeting or exceeding a high confidence level for one or more types of activities. After the model has undergone an appropriate amount of training, the model can be used for heart rate prediction if the actual measurement confidence level and/or signal quality measurements for heart rate are low and/or fail to meet a minimum confidence level threshold.

Since devices are usually used by a single user most of the time (or a single user profile is logged into a device at one time), personalized heart rate prediction can be implemented with the devices, methods, and related systems described herein. As a non-limiting example, a method to dynamically predict heart rate for display or reporting to a user or other person as described herein can fill in gaps in reliable heart rate monitoring caused by low signal quality regions due to a subject user's physiological properties. This personalized heart rate prediction can be achieved, by training a model by capturing heart rate and/or movement data using sensors over many iterations of performing an activity, such as running, walking, rowing, biking/cycling, or other activities where heart rate may become elevated due to exercise. In one example, by tracking signal quality of a PPG monitor and detecting high and low signal quality regions, using a heart rate confidence level property of a heart rate measurement module, for different types of activities, and using the measurements with high quality and high confidence for predicting a measurement where the signals have low quality and/or a low confidence level. The type of activity can be determined by calculating motion frequency and accelerometer density, and, based on the values, classifying the subject's mode of activity, such as resting, walking, running, biking, or others. Further, to build the prediction model or algorithm, two-dimensional (2d) lookup tables can be defined for each activity that include heart rate, motion frequency, and accelerometer density. It should be understood that instead of tables, functions and/or sets of rules and/or machine learning models can be used. In various aspects, the input number and type of these tables, models and/or functions can vary. For example, single input, dual input (e.g., motion frequency and accelerometer norm density), or other numbers of inputs can be used. Additionally, such lookup tables include a maximum or mean expected heart rate in each activity for the subject user. For instance, generally, lookup tables can be accurately mapped when a user performs consistent activities of the same sort with similar motion frequency and similar density, which will typically yield similar heart rate values - e.g., daily walking, running, rowing, or others. Moreover, the tables can be populated by filling in heart rate values calculated in high signal quality regions and interpolating for each activity. Then, once a certain amount of data has been included in the lookup tables (e.g., coverage of the lookup table is greater than a certain threshold, such as greater than 70%), the lookup table is prepared for use in heart rate prediction to be displayed and/or reported in low signal quality regions. For example, the system, device, and method can provide a maximum or mean expected heart rate value via the lookup table for a determined activity based on correlation with motion frequency and accelerometer norm and/or density values. Additionally, the system, device, and method may apply auto-regressive filtering to base the prediction on recent high quality/confidence measurements.

As an example, for a subject user A, a maximum heart rate could be 164 BPM with 1.2 Hz motion frequency and 4 g accelerometer norm (LP filtered) for a running activity. An initial heart rate value may be the last high confident measured heart rate. A final target of an auto-regressive filter can be the maximum heart rate (2d lookup table output). An auto-regressive or exponential smoothing filtering coefficient can be determined and/or optimized for each subject (e.g., 7/8, 15/16, 31/32, 63/64, 127/128, 0.875, 0.9375, 0.96875, 0.984375, or others).

In various aspects, movement data corresponding to user movements is captured by movement sensors can be used to determine acceleration density, which can be a sum, norm, cubic, quaternion, or other function of different combinations of accelerometer x, y, z axes measurements. This information can be used in methods for determining maximum or mean expected heart rate during a particular activity by characterizing a movement activity and storing in association with and/or using 2d lookup tables with features of motion frequency and accelerometer density. Applicable linear and nonlinear functions can include Maximum_heart_rate: a * motion_frequency + b * accelerometer_density; Maximum_heart_rate: a + b * e^(c * motion_frequency + d * accelerometer_density). As mentioned above, machine learning models can also be employed. Post-processing can include various types, degree of FIR/IIR filtering, Kalman filtering/tracking, and others. As also mentioned above, in different aspects, the number of inputs can vary.

Additional advantages can be realized in some aspects, including better monitoring and prediction of HR related statistics such as VO2 max (i.e., the maximum oxygen consumption during exercise), recovery, and others.

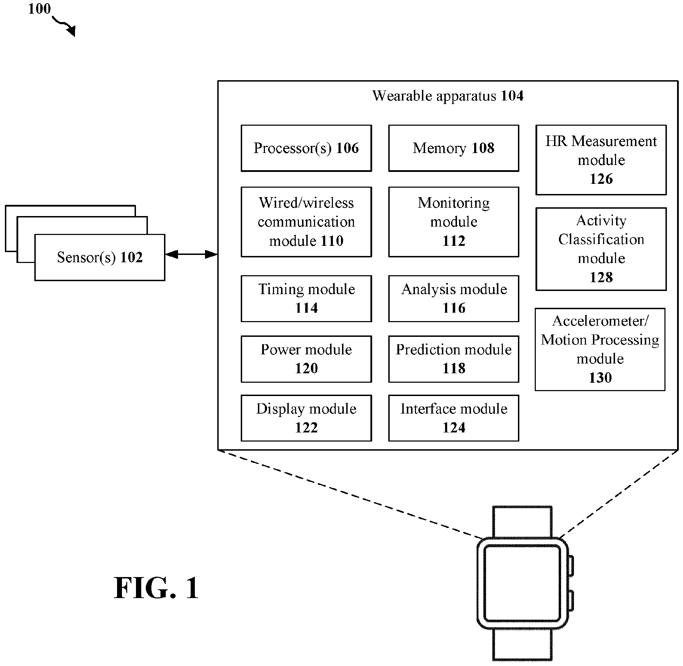

FIG. 1 is a diagram 100 illustrating an example of a heart rate prediction architecture. As shown, a heart rate prediction architecture can generally include one or more sensor(s) 102 communicatively coupled with a wearable apparatus 104. In various aspects sensor(s) 102 can be wearable and can be stand-alone and/or integrated into and/or coupled with various devices or apparatuses. Examples of the wearable device or apparatus 104 includes, but is not limited to, a wrist band, a bracelet, an anklet, a ring, a smart watch, a chest band, an arm band, a leg band, an ear bud or headphones, goggles, glasses, or any other type of wearable device. Examples of the one or more sensors 102 can include, but are not limited to, one or more of visual sensors such as opto-electrical sensors; audio sensors such as receivers, monitors, transducers, microphones, ultrasonic, or other sonic sensors; combination audio-visual sensors such as infrared, ultraviolet, color, light, or other photonic sensors; movement or position sensors such as tilt sensors, accelerometers (e.g., three-axis (3-axis) accelerometers), or gyroscopes; vibration sensors; proximity sensors; current sensors; thermal sensors such as thermistors or other temperature sensors; pressure sensors; moisture or humidity sensors; olfactory sensors; chemical sensors; temperature sensors; touch sensors; strain sensors; weight sensors; heart beat sensors; and timers; electrocardiogram (ECG) sensors; and/or any other type of sensor configured to provide parameters relating to determining heart rate, type of activity, and user motion. In some aspects one or more of the sensors 102 can be communicatively coupled with one or more other sensors 102, while in some aspects the one or more sensors 102 can be communicatively coupled only with the wearable apparatus 104. Communicative coupling can be wireless and/or wired in various aspects. In aspects implementing wireless communicative couplings, one or more wireless links or connections can be maintained by one or more wireless receivers, transmitters, and/or transceivers that is included or implemented with the one or more sensors 102. In some aspects one or more processors and/or controllers can be coupled with the sensor(s) 102 as well.

When a host application performs heart rate prediction, it can accomplish this task by one or more processors 106 using heart rate data gathered by monitoring heart rate and/or other data related to physiological functioning of a user that is captured, tracked, and/or recorded by the one or more sensors 102. In various aspects the host application can issue a command to start a predefined monitoring process, stored in non-transitory computer readable memory (e.g., onboard or coupled with the one or more memory 108) and executed alone or in combination by one or more of the processors 106.

Monitoring can be accomplished by one or more processors 106 capturing, tracking, and/or recording data via the one or more sensors 102 and storing data in the one or more memories 108. Capturing, tracking, and/or recording data can be accomplished passively and/or actively, based on capabilities of the one or more sensors 102 according to one or more programs and/or instructions stored in one or more memory 108 and executed by one or more processors 106. For example, a user of the wearable apparatus 104 can cause one or more processors 106 of the wearable apparatus 104 to turn on and run one or more data monitoring programs using interface module 124, which can manage user interfacing with wearable apparatus 104 via one or more interfaces, such as a touchscreen, keypad, keyboard, audio, visual, combined audio-visual and/or other interfaces. A display module 122 can cause a screen or touchscreen of the wearable apparatus 104 to show data, graphics, and/or other information to the user, such as heart rate monitoring data and/or heart rate monitoring diagram(s).

In some aspects, once the user has engaged a monitoring process, the one or more processor(s) 106 can cause the monitoring module 112 to send one or more commands to the one or more sensor(s) 102 and/or receive data from the one or more sensor(s) 102 via a wired and/or wireless communication module 110. This data can include heart rate monitoring data that can then be stored in the one or more memory 108. Data stored in the one or more memory 108 can be analyzed via the analysis module 116 and analyzed data can be stored in the one or more memory 108. Prediction module 118 can use the data and/or analyzed data stored in the one or more memory to generate predictions and fill in holes in the data or analyzed data that may be corrupt, incomplete, or otherwise unreliable. Analysis module 116 can determine whether data is corrupt, incomplete, or otherwise unreliable in various aspects based on whether it matches or meets expected data characteristics. This matching or meeting can be accomplished via comparison with thresholds to determine if measured data is outside of expected ranges or otherwise appears to be unrealistic for human performance or past user performance based on historical data.

In some aspects, a heart rate (HR) measurement module 126 can be included and can measure heart rate of the user as indicated by data captured by the one or more sensor(s) 102. Heart rate measurement module 126 can measure PPG and accelerometer signals; determine the reliability of the measured heart rate, including a confidence level; detect signal quality (based on confidence level); help in training the tables, models, functions; and determine if the confidence level is low (based on confidence level); and utilize the table, model, function to predict heart rate.

An activity classification module 128 can be used to determine what type of activity a user is engaged in, based on data captured by the one or more sensor(s) 102. For example, movement profiles for different activities (e.g., rowing, running, walking, swimming, biking/cycling, or others) are different based on the way a person moves and can generate a unique profile from user to user based on the unique ways a person moves their body. This can be a form of classification of activities and can be used in training different tables, models, functions, or others for different activities.

Measuring movements can occur using an accelerometer/motion processing module 130 based on accelerometers, gyroscopes, and/or other movement and/or position sensors 102 and can provide a comprehensive understanding of the types of movement a user engages in and be used to generate better heart rate analysis and/or prediction results.

In various aspects a modeling methodology can include calculating a motion frequency and accelerometer density, and classifying the subject user's activity based on such determination. Classification can be in the form of activity, such as resting, walking, running, biking/cycling, or others. 2d lookup tables can be defined for each activity which takes motion frequency and accelerometer density and gives the maximum heart rate in that activity for the subject user. The lookup table can be filled with the heart rate values calculated in high signal quality regions and interpolated for each activity. After a certain amount of data is fed to the lookup tables (e.g., coverage of the lookup table meets or exceeds a certain threshold, such as > 70%), it can be used for heart rate prediction to be reported in low signal quality regions. Models can be trained for prediction and in some aspects are not restricted solely to lookup tables. Some models can use a machine learning model or a linear/non-linear function etc.

In post processing, lookup tables can give a maximum heart rate value for an activity with the associated motion frequency and accelerometer density values. For example, for a subject user performing a running activity, a maximum heart rate is 164 BPM with 1.2 Hz motion frequency and 4 g accelerometer norm (LP filtered). The predicted heart rate can then be subjected to auto-regressive filtering. An initial heart rate value can be a last high confident measured heart rate. A final target of the auto-regressive filter can be a maximum heart rate (e.g., based on a 2d lookup table output). Additionally, an auto-regressive or exponential smoothing filtering coefficient can be determined for each subject user (e.g., 7/8, 15/16, 31/32, 63/64, 127/128, 0.875, 0.9375, 0.96875, 0.984375, or others).

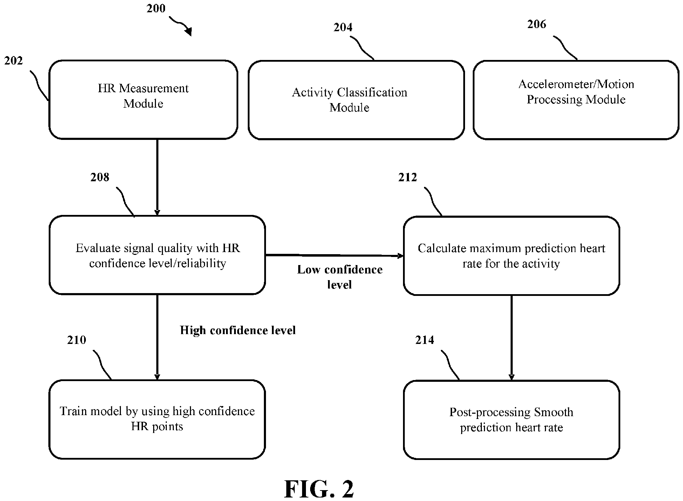

FIG. 2 is a flowchart 200 illustrating an example of a method of monitoring and predicting a heart rate of a user. As shown, one or more of a HR measurement module 202, activity classification module 204, accelerometer/motion processing module 206, and/or others can run at particular times and/or continuously based on user commands executed by one or more processors 106. These modules can generate data and in block 208 this data can be evaluated based on, at least, signal quality to determine a heart rate confidence level and/or reliability of the data. For example, in some aspects, confidence levels can be split into various tiers, levels, or ranges. For example, but not to be construed as limiting, a range of 0% - 25% confidence level can be indicated according to low signal quality as indicated by measured and/or monitored data and analyzed by the prediction apparatus. Also, for example, but not to be construed as limiting, a range of 75% - 100% confidence level can be indicated according to high signal quality as indicated by measured and/or monitored data and analyzed by the prediction apparatus. These ranges may be configurable, e.g., different and/or wider and/or smaller ranges, by an implementer of these aspects depending on how the implementer chooses to define low and high quality signals/data.

Data that has been evaluated and indicates a high confidence level according to the evaluation at block 208 can be used at block 210 to train one or more heart rate prediction models. This can be performed by using the measuring heart rate values in correlation with motion frequency, accelerometer density, timing, and/or other data, according to one or more lookup tables. In some aspects, lookup tables can be two dimensional, three dimensional, or both. Lookup tables can also be charts, graphs, diagrams, or others in various aspects. In some aspects, machine learn models, functions, or other methods can be used to correlate data and train the model(s). High confidence level HR measurements can be displayed to the user. In other words, the device can report the measurement if it is considered reliable, and the device can use it directly in displaying to the user without modifying it according to a model or post-processing such as smoothing, which may occur when a confidence level is not high (i.e., when the confidence level is low).

Data that has been evaluated and indicates a low confidence level according to the evaluation at block 208 can cause the method to move to block 212. At block 212 where low signal quality that corresponds to a low confidence level has occurred, motion frequency, position, accelerometer density, timing and/or other data can be inputted into or otherwise used by a developed and/or trained model. This developed and/or trained model can yield a maximum or mean expected prediction heart rate for an activity that it appears that the user has been engaging in. At block 214, post-processing can occur which smooths a prediction heart rate. This can be accomplished by initializing a FIR/IIR filter and/or any kind of filter for smoothing with the last confidently measured heart rate value according to one or more metrics, as determined by comparison with thresholds and/or ranges. A maximum and/or expected predicted heart rate can be used to filter and smoothly regress the predicted heart rate data to generate a realistic heart rate profile (e.g., according to the user's historical heart rate activity when engaging in the same and/or similar activity at the same and/or similar exertion level).

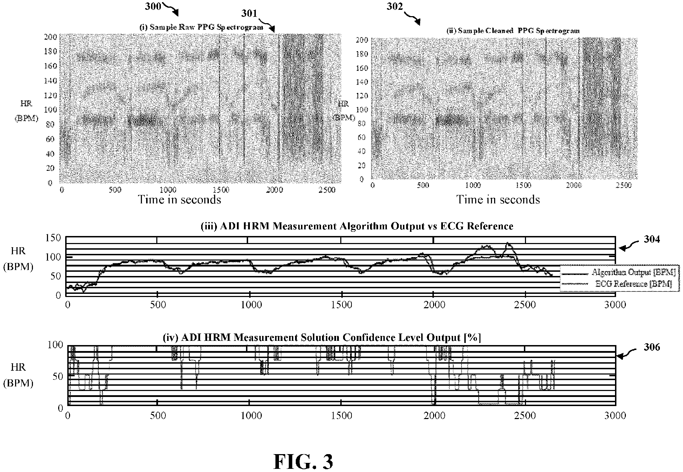

FIG. 3 is a set of diagrams illustrating an example of a PPG signal power spectrogram 300, raw PPG spectrogram diagram 301, motion compensated PPG power spectrogram diagram 302, heart rate monitoring measurement model algorithm with heart rate output and comparison with an electrocardiogram-based reference signal diagram 304, and heart rate measurement model algorithm confidence level output diagram 306. Each of diagrams 300, 302, 304, and 306 are related and the timelines of diagrams 304, 302, and 300 match, although the scale and length of the axes are slightly different. For example, diagram 306 shows confidence intervals associated with data shown in diagram 304, which shows a model of the data set of diagram 302 and diagram 301.

As shown, a PPG signal power spectrogram diagram 300 can be shown as a graph of heart rate measured in beats per minute versus time in seconds. In an interval training scenario, this can include intervals of rising and/or sustained high heart rates with short intervals of quickly reduced heart rate. Also shown are locations of corrupted data 301 where the monitoring device (e.g., sensor(s)) may not have captured high quality data for one of any number of reasons (e.g., loose fit, sweat interference, jostling or other movements causing the sensor(s) to not remain in a fixed monitoring location with respect to the user's body location, heat, or others) and which create static, noise or otherwise unreliable and unusable sections of data. While this can occur at any time during an activity, it may be more likely to occur as time goes on with respect to particular activities where the user is performing high levels of physical exertion. As shown, locations of corrupted data 301 can include a wide range for a set of samples at a same or similar time, e.g., the data between about 2250-2500 seconds in the diagram.

A motion compensated PPG power spectrogram diagram 302 can be shown as a graph of heart rate measured in beats per minute versus time in seconds. It should be understood that the motion compensated PPG power spectrogram diagram 302 illustrates an example of the PPG signal power spectrogram diagram 300 after the signal has been cleaned and/or compensated via smoothing according to one or more smoothing operations. In some aspects, these smoothing operations can include using high confidence data as a substitute for lower quality data. In an interval training scenario, this can include intervals of rising and/or sustained high heart rates with short intervals of quickly reduced heart rate. With relatively short intervals of corrupted or unreliable data, smoothing can be used to clean up the measured data as compared to expected data. Thus, a number of locations in the measured curves are smoothed and cleaned as compared to the sample raw motion compensated PPG power spectrogram diagram 300.

A heart rate monitoring algorithm with heart rate output and an electrocardiogram golden heart rate reference diagram 304 can be shown as heart rate in beats per minute versus time in seconds. The heart rate monitoring algorithm with heart rate output and an electrocardiogram golden heart rate reference diagram 304 shown is related to diagram 300 and diagram 302 and the timelines of diagrams 304, 302, and 300 match, although the scale and length of the axes are slightly different. Diagram 304 shows the same model of the data set of diagram 302 and diagram 300, a heart rate monitoring or measurement algorithm as a modeled heart rate versus time. Another line shows a golden reference for an ECG reference of heart rate versus time.

A heart rate measurement confidence levels provided by a heart rate monitoring algorithm diagram 306 can be shown as heart rate in beats per minute versus time in seconds. The heart rate monitoring algorithm diagram 306 shown is related to diagram 304, diagram 302, and diagram 300 and the timelines of diagrams 306, 304, 302, and 300 match, although the scale and length of the axes are slightly different. Where a monitored or measured heart rate deviates from an algorithm model or ECG reference, a percentage difference can be calculated. Based on the percentage difference calculation, a confidence level output can be assigned. Thresholds, ranges, or other metrics can be used and when percentage difference or deviation from a reference is too great or outside an acceptable range, other operations can be performed. These operations can include smoothing, filling in data based on expected data, or others.

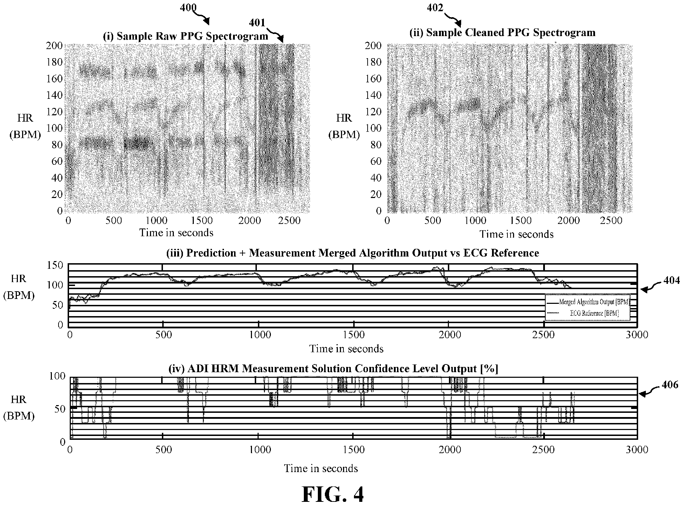

FIG. 4 is a set of diagrams illustrating an example of a PPG signal power spectrogram diagram 400, motion compensated PPG power spectrogram diagram 402, heart rate monitoring algorithm with heart rate output using an optical measurement enhanced with a heart rate prediction model and an electrocardiogram heart rate reference diagram 404, and heart rate measurement confidence levels provided by a heart rate monitoring algorithm diagram 406. Each of diagrams 400, 402, 404, and 406 are related and the timelines of diagrams 404, 402, and 400 match, although the scale and length of the axes are slightly different. For example, diagram 406 shows confidence intervals associated with data shown in diagram 404, which shows a model of the data set of diagram 402 and diagram 400.

As shown, a PPG signal power spectrogram diagram 400 can be shown as a graph of heart rate measured in beats per minute versus time in seconds. In an interval training scenario, this can include intervals of rising and/or sustained high heart rates with short intervals of quickly reduced heart rate. Also shown are locations of corrupted data 401 where the monitoring device (e.g., sensor(s)) may not have captured high quality data for one of any number of reasons (e.g., sweat interference, jostling or other movements causing the sensor(s) to not remain in a fixed monitoring location with respect to the user's body location, heat, or others) and which create static, noise or otherwise unreliable and unusable sections of data. While this can occur at any time during an activity, it may be more likely to occur as time goes on with respect to particular activities where the user is performing high levels of physical exertion. As shown, locations of corrupted data 401 can include a wide range for a set of samples at a same or similar time, e.g., the data between about 2250-2500 seconds in the diagram.

A motion compensated PPG power spectrogram diagram 402 can be shown as a graph of heart rate measured in beats per minute versus time in seconds. In an interval training scenario, this can include intervals of rising and/or sustained high heart rates with short intervals of quickly reduced heart rate. With relatively short intervals of corrupted or unreliable data, smoothing can be used to clean up the measured data as compared to expected data. Thus, a number of locations in the measured curves are smoothed and cleaned as compared to the sample raw motion compensated PPG power spectrogram diagram 402.

A heart rate monitoring algorithm with heart rate output and an optical measurement enhanced with a heart rate prediction model and an electrocardiogram heart rate reference diagram 404 can be shown as heart rate in beats per minute versus time in seconds. A heart rate prediction and measurement or monitoring merged algorithm can show a modeled heart rate merged with a predicted heart rate versus time. An ECG reference of heart rate versus time.

A heart rate measurement confidence levels provided by a heart rate monitoring algorithm diagram 406 can be shown as heart rate in beats per minute versus time in seconds. Where a merged predicted and monitored or measured heart rate deviates from an algorithm model or ECG reference, a percentage difference can be calculated. Based on the percentage difference calculation, a confidence level output can be assigned. Thresholds, ranges, or other metrics can be used and when percentage difference or deviation from a reference is too great or outside an acceptable range, other operations can be performed. These operations can include smoothing, filling in data based on expected data, or others.

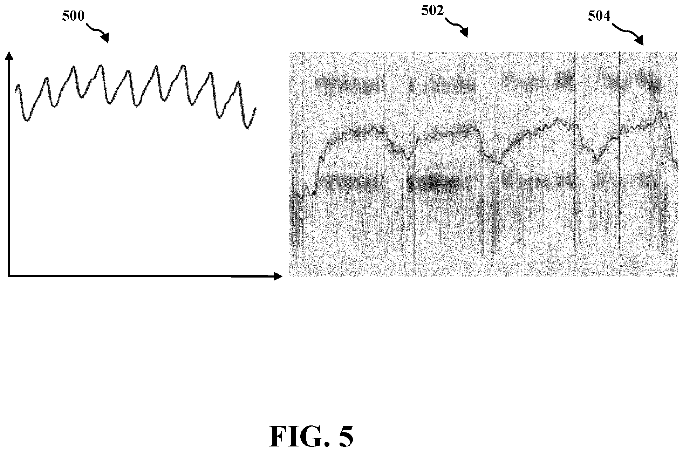

FIG. 5 is a set of diagrams illustrating an example of heart rate versus time diagram 500 and raw PPG power spectrogram diagram 502. As shown, in an interval training scenario heart rate versus time diagram 500, this can include intervals of rising and/or sustained high heart rates with short intervals of quickly reduced heart rate. Also shown in the raw PPG power spectrogram diagram 502 are locations of corrupted data 504 where the monitoring device (e.g., sensor(s)) may not have captured high quality data for one of any number of reasons (e.g., sweat interference, jostling or other movements causing the sensor(s) to not remain in a fixed monitoring location with respect to the user's body location, heat, or others) and which create static, noise or otherwise unreliable and unusable sections of data. While this can occur at any time during an activity, it may be more likely to occur as time goes on with respect to particular activities where the user is performing high levels of physical exertion.

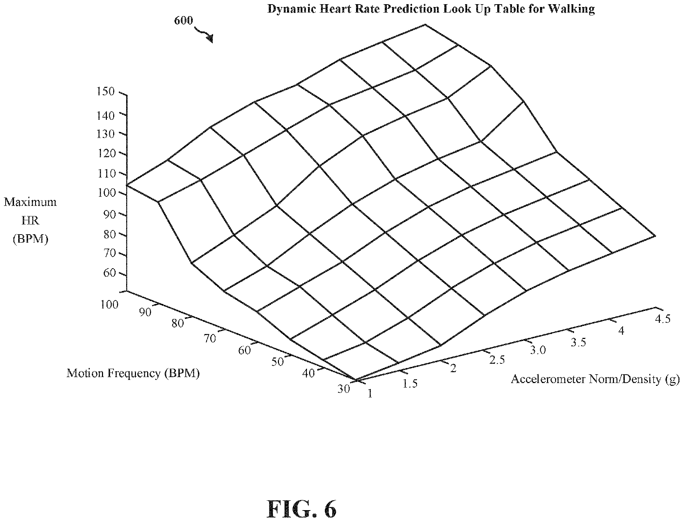

FIG. 6 is a diagram 600 illustrating an example of a dynamic heart rate prediction look up graph for walking. As shown, for walking a maximum heart rate in beats per minute can be cross referenced with an accelerator norm/density in gravity (g) and motion frequency in beats per minute. As such, when data from a heart rate sensor is unreliable, corrupted, or otherwise outside a normal realm of possibility, associated measurements taken at the same time by an accelerometer and/or motion frequency can be cross referenced to determine a predicted maximum heart rate for walking based on the looked-up values. For accelerometer norm (e.g., which is Euclidean norm ACC_norm = sqrt(x^2 + y^2 + z^2)), with unit g. The accelerometer density can be g/second or g/min etc. Acceleration density can be average acceleration (absolute) values over certain time.

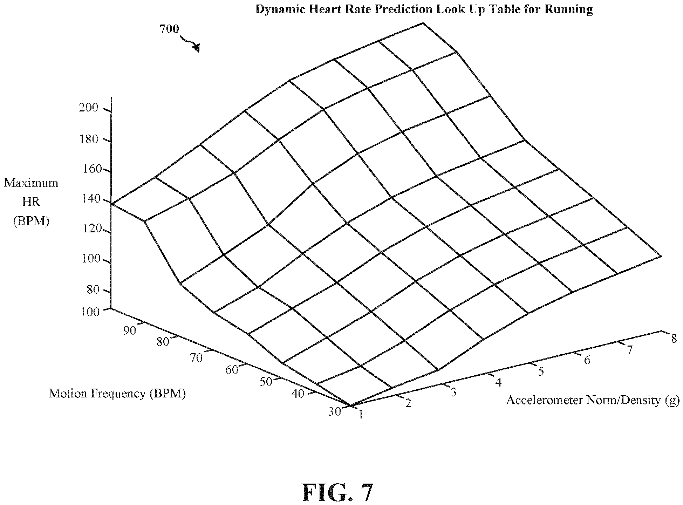

FIG. 7 is a diagram 700 illustrating an example of a dynamic heart rate prediction look up graph for running. As shown, for running a maximum heart rate in beats per minute can be cross referenced with an accelerator norm/density in g and motion frequency in beats per minute. As such, when data from a heart rate sensor is unreliable, corrupted, or otherwise outside a normal realm of possibility, associated measurements taken at the same time by an accelerometer and/or motion frequency can be cross referenced to determine a predicted maximum heart rate for running based on the looked-up values.

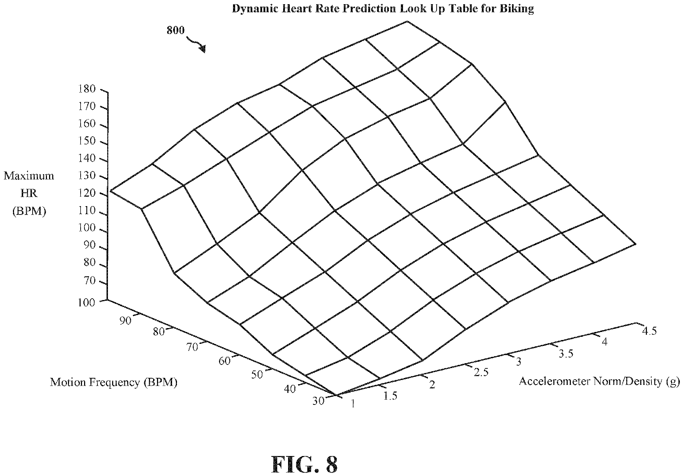

FIG. 8 is a diagram 800 illustrating an example of a dynamic heart rate prediction look up graph for biking/cycling. As shown, for biking/cycling a maximum heart rate in beats per minute can be cross referenced with an accelerator norm/density in g and motion frequency in beats per minute. As such, when data from a heart rate sensor is unreliable, corrupted, or otherwise outside a normal realm of possibility, associated measurements taken at the same time by an accelerometer and/or motion frequency can be cross referenced to determine a predicted maximum heart rate for biking/cycling based on the looked-up values.

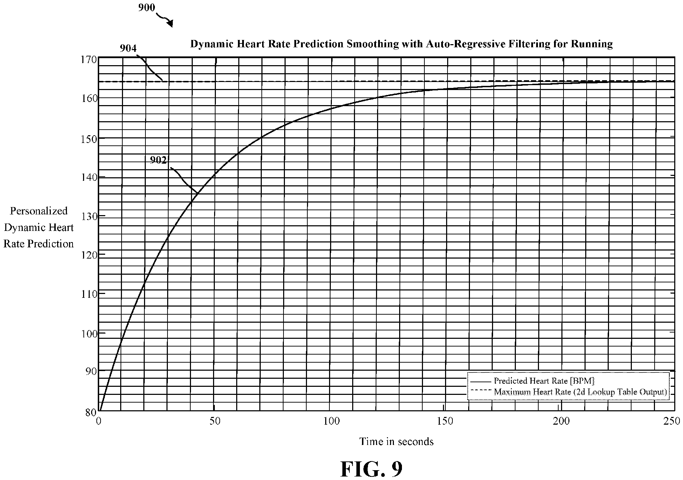

FIG. 9 is a diagram 900 illustrating dynamic heart rate prediction smoothing with auto-regressive filtering for running. As shown, a personalized dynamic heart rate prediction in beats per minute versus time (e.g., seconds) in a sample can be graphed. A predicted heart rate can be a generally logarithmic curve for running, as indicated by the solid curved line 902. A maximum heart rate based on a two-dimensional lookup table for running is indicated by the straight dashed line 904.

In various aspects, when a captured signal is unreliable, for instance because sensors 102 are unable to capture a clear signal due to excessive noise from movement, sweat, or other interference problems, smoothing can be performed by using the maximum heart rate value according to a 2d lookup table as accessed by the one or more processors 106, checking the last time t-1 when a high confidence measurement was captured, and determining where on the solid curved line 902 the heart rate would be if the signal was high quality.

A last measured heart rate with high confidence can be located at the origin of the diagram. In the example, a maximum heart rate at 80bpm (1.33 Hz) motion frequency and 5 g accelerometer norm (LP filtered) for a running activity are shown for the subject user.

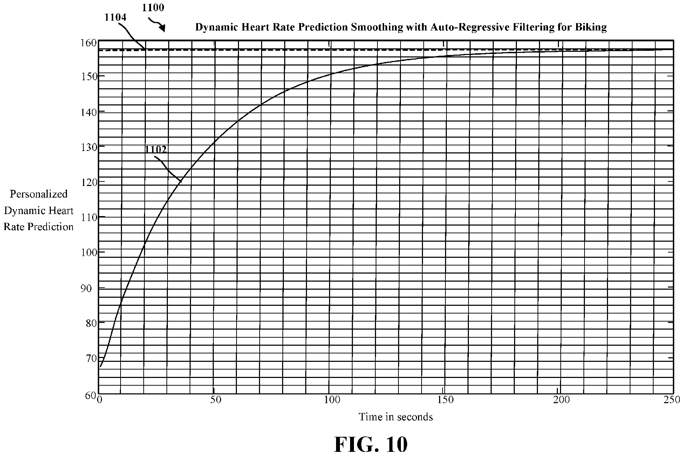

FIG. 10 is a diagram 1100 illustrating dynamic heart rate prediction smoothing with auto-regressive filtering for biking/cycling. As shown, a personalized dynamic heart rate prediction in beats per minute versus time (e.g., seconds) in a sample can be graphed. A predicted heart rate can be a generally logarithmic curve for biking/cycling, as indicated by the solid curved line 1102. A maximum heart rate based on a two-dimensional lookup table for biking/cycling is indicated by the straight dashed line 1104.

In various aspects, when a captured signal is unreliable, for instance because sensors 102 are unable to capture a clear signal due to excessive noise from movement, sweat, or other interference problems, smoothing can be performed by using the maximum heart rate value according to a 2d lookup table as accessed by the one or more processors 106, checking the last time t-1 when a high confidence measurement was captured, and determining where on the solid curved line 1102 the heart rate would be if the signal was high quality.

A last measured heart rate with high confidence can be located at the origin of the diagram. In the example, a maximum heart rate at 80bpm (1.33 Hz) motion frequency and 4 g accelerometer norm (LP filtered) for a biking/cycling activity are shown for the subject user.

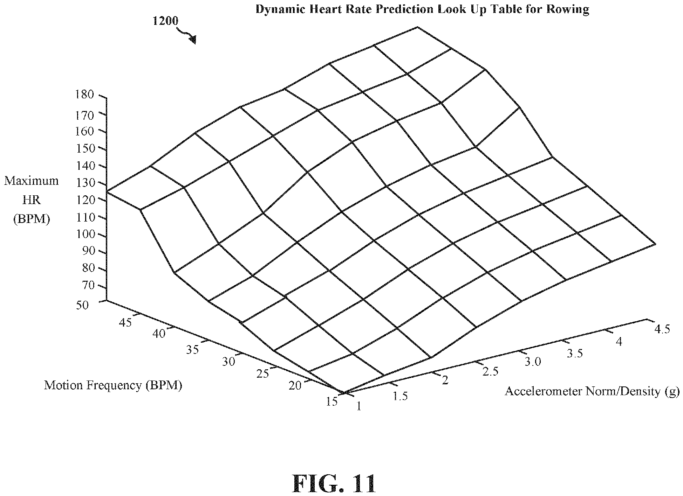

FIG. 11 is a diagram 1200 illustrating an example of a dynamic heart rate prediction look up graph for rowing. As shown, for rowing a maximum heart rate in beats per minute can be cross referenced with an accelerator norm/density in g and motion frequency in beats per minute. As such, when data from a heart rate sensor is unreliable, corrupted, or otherwise outside a normal realm of possibility, associated measurements taken at the same time by an accelerometer and/or motion frequency can be cross referenced to determine a predicted maximum heart rate for rowing based on the looked-up values.

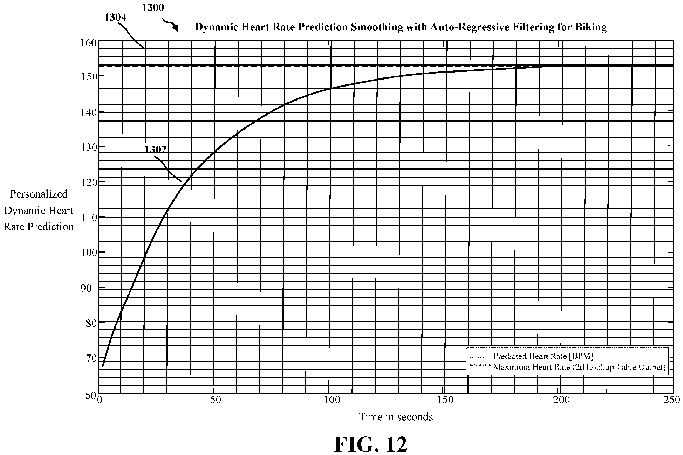

FIG. 12 is a diagram 1300 illustrating dynamic heart rate prediction smoothing with auto-regressive filtering for rowing. As shown, a personalized dynamic heart rate prediction in beats per minute versus time (e.g., seconds) in a sample can be graphed. A predicted heart rate can be a generally logarithmic curve for rowing, as indicated by the solid curved line 1302. A maximum heart rate based on a two-dimensional lookup table for rowing is indicated by the straight dashed line 1304.

A last measured heart rate with high confidence can be located at the origin of the diagram. In the example, a maximum heart rate at 40bpm (0.66 Hz) motion frequency and 3.5 g accelerometer norm (LP filtered) for a rowing activity are shown for the subject user.

In various aspects, when a captured signal is unreliable, for instance because sensors 102 are unable to capture a clear signal due to excessive noise from movement, sweat, or other interference problems, smoothing can be performed by using the maximum heart rate value according to a 2d lookup table as accessed by the one or more processors 106, checking the last time t-1 when a high confidence measurement was captured, and determining where on the solid curved line 1302 the heart rate would be if the signal was high quality.

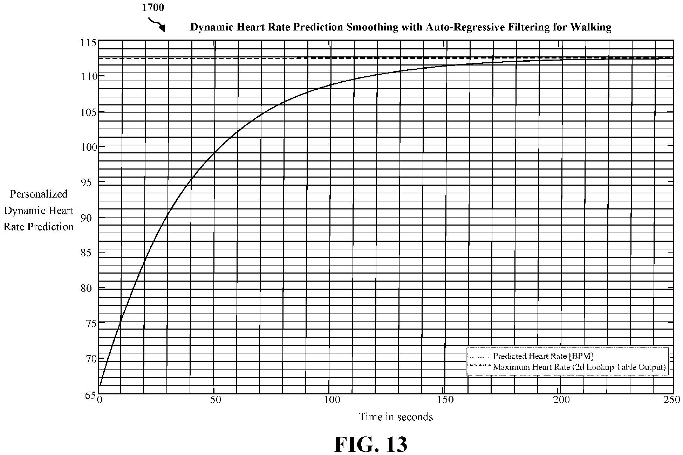

FIG. 13 is a diagram 1700 illustrating dynamic heart rate prediction smoothing with auto-regressive filtering for walking. As shown, a personalized dynamic heart rate prediction in beats per minute versus time (e.g., seconds) in a sample can be graphed A predicted heart rate can be a generally logarithmic curve for walking, as indicated by the solid curved line 1102. A maximum heart rate based on a two-dimensional lookup table for walking is indicated by the straight dashed line 1104.

In various aspects, when a captured signal is unreliable, for instance because sensors 102 are unable to capture a clear signal due to excessive noise from movement, sweat, or other interference problems, smoothing can be performed by using the maximum heart rate value according to a 2d lookup table as accessed by the one or more processors 106, checking the last time t-1 when a high confidence measurement was captured, and determining where on the solid curved line 1702 the heart rate would be if the signal was high quality.

A last measured heart rate with high confidence can be located at the origin of the diagram. In the example, a maximum heart rate at 70bpm (1.16 Hz) motion frequency and 3.5 g accelerometer norm (LP filtered) for a walking activity are shown for the subject user.

As described herein, determination of accurate and failed HR measurements can be important in various aspects. Accurate points are determined with the HRM confidence level > threshold (e.g., for last 5 seconds, 10 seconds, 15 seconds, or other interval based on application, use case, methodology, hardware, sensor/signal, or others). Failed points are determined with HRM confidence level == 0 (for last 5 seconds, 10 seconds, 15 seconds, or other interval based on application, use case, methodology, hardware, sensor/signal, or others) or <= 25% or another comparison in various aspects. This comparison can vary based on application, use case, methodology, hardware, sensor/signal, or others. Low signal to noise ratio (SNR), which can be challenging, data points are included. Detection of failed regions is possible by using HR confidence level (if ==0), or other interval based on application, use case, methodology, hardware, sensor/signal, or others) or <= 25% or another comparison in various aspects. This comparison can vary based on application, use case, methodology, hardware, sensor/signal, or others.

**Table 1 - Walking (example)**

| Metrics | 1^{st} Elimination | 2^{nd} Elimination | 3^{rd} Elimination | 4^{th} Elimination | Default |
|---|---|---|---|---|---|
| 5 bpm accuracy % | 98.2 | 95.51 | 92.52 | 90.64 | 77.52 |
| Reporting coverage % | 10 | 20 | 40 | 50 | 100 |

**Table 2 - Running (example)**

| Metrics | 1^{st} Elimination | 2^{nd} Elimination | 3^{rd} Elimination | 4^{th} Elimination | Default |
|---|---|---|---|---|---|
| 5 bpm accuracy % | 98.36 | 96.73 | 94.55 | 92.28 | 77.6 |
| Reporting coverage % | 10 | 20 | 40 | 50 | 100 |

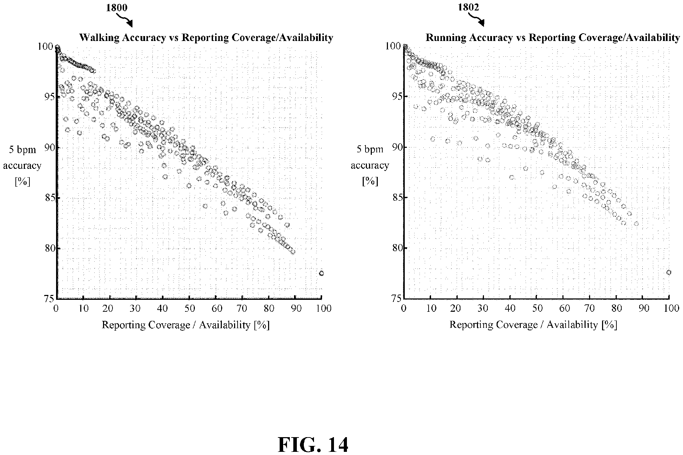

FIG. 14 is a set of diagrams illustrating walking accuracy versus reporting coverage/availability in a diagram 1800 and running accuracy versus reporting coverage/availability in a diagram 1802.

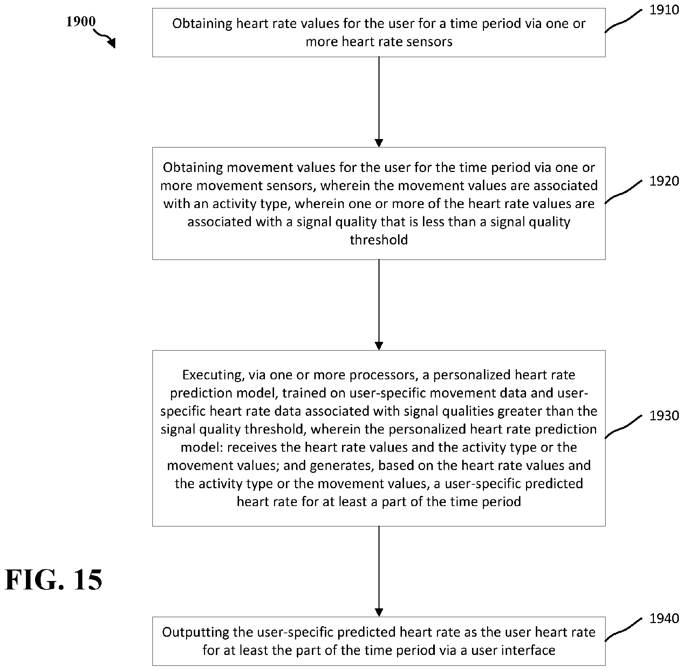

FIG. 15 is a flowchart 1900 illustrating a method for determining a personalized heart rate for a user of a heart rate prediction apparatus.

At block 1910, method 1900 includes obtaining heart rate values for the user for a time period via one or more heart rate sensors. For example, in an aspect the one or more processors 106 are configured to or comprise means for causing the one or more heart rate sensors 102 to capture a heart rate reading that is stored in one or more memories 108.

At block 1920, method 1900 includes obtaining movement values for the user for the time period via one or more movement sensors, wherein the movement values are associated with an activity type, wherein one or more of the heart rate values are associated with a signal quality that is less than a signal quality threshold. For example, in an aspect the one or more processors 106 are configured to or comprise means for causing the one or more movement sensors 102 to capture a movement reading that is stored in one or more memories 108.

At block 1930, method 1900 includes executing, via one or more processors, a personalized heart rate prediction model, trained on user-specific movement data and user-specific heart rate data associated with signal qualities greater than the signal quality threshold, wherein the personalized heart rate prediction model: receives the heart rate values and the activity type or the movement values; and generates, based on the heart rate values and the activity type or the movement values, a user-specific predicted heart rate for at least a part of the time period. For example, in an aspect the one or more processors 106 are configured to or comprise means for executing the personalized heart rate prediction model and generating a user-specific predicted heart rate for at least a part of the time period. Instructions and/or a program for the personalized heart rate prediction model can be stored in one or more memories 108.

At block 1940, method 1900 includes outputting the user-specific predicted heart rate as the user heart rate for at least the part of the time period via a user interface. For example, in an aspect the one or more processors 106 are configured to or comprise means for outputting, via the user interface, the user-specific predicted heart rate as the user heart rate for at least the part of the time period.

In an alternative or additional aspect, method 1900 may further include wherein the activity type comprises a non-resting activity type.

In an alternative or additional aspect, method 1900 may further include wherein the non-resting activity type is running, walking, rowing, or biking.

In an alternative or additional aspect, method 1900 may further include wherein obtaining heart rate values further comprises obtaining a heart rate signal having a plurality of frequency components, and wherein executing the personalized heart rate prediction model further comprises selecting one of the plurality of different frequency components as corresponding to the heart rate value.

In an alternative or additional aspect, method 1900 may further include wherein the personalized heart rate prediction model is trained on user-specific movement data and user-specific heart rate data associated with signal qualities greater than the signal quality threshold by storing the user-specific movement data and user-specific heart rate data in a lookup table associated with the activity type.

In an alternative or additional aspect, method 1900 may further include wherein the lookup table is generated based on a plurality of user-specific movement data and user-specific heart rate data for the activity type.

In an alternative or additional aspect, method 1900 may further include wherein the activity type is determined based on motion frequency and density.

In an alternative or additional aspect, method 1900 may further include wherein the one or more movement sensors comprise a 3-axis accelerometer and a gyroscope.

In an alternative or additional aspect, method 1900 may further include wherein the one or more heart rate sensors comprise a photoplethysmography (PPG) sensor.

In an alternative or additional aspect, method 1900 may further include evaluating signal quality with a confidence interval for the measured heart rate for a period of time. For example, in an aspect the one or more processors 106 are configured to or comprise means for evaluating, via comparing with one or more thresholds and/or intervals, signal quality to determine a signal quality confidence level.

In an alternative or additional aspect, method 1900 may further include generating the user-specific predicted heart rate for at least a part of the time period, when the evaluated signal quality has a low confidence interval, the one or more processors are further configured to calculate a maximum prediction heart rate for the activity and perform post-processing smoothing prediction for the heart rate. For example, in an aspect the one or more processors 106 are configured to or comprise means for calculating a maximum prediction heart rate for the activity and performing post-processing smoothing prediction for the heart rate.

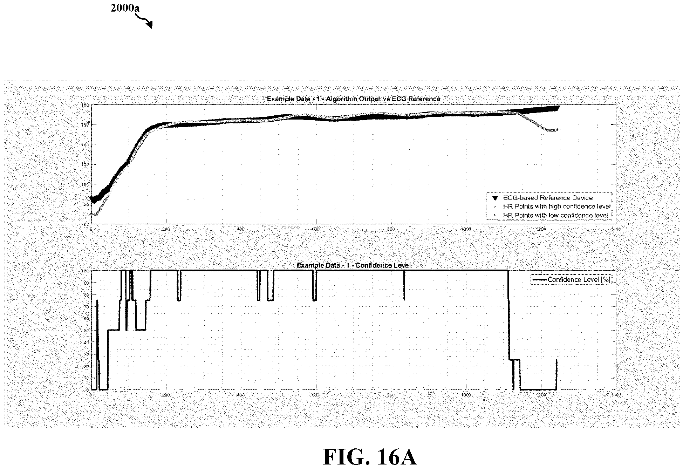

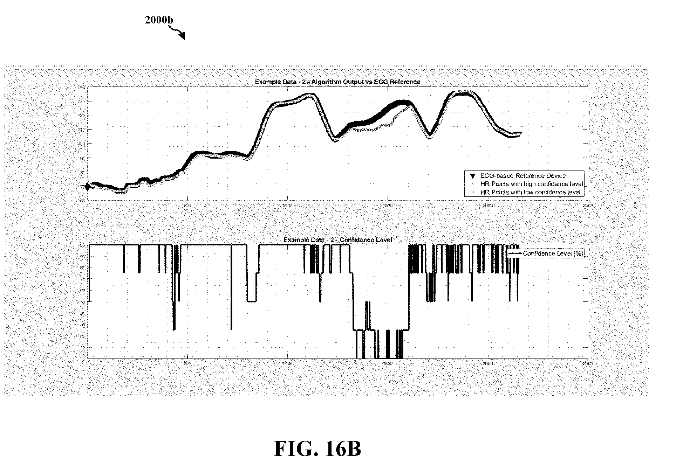

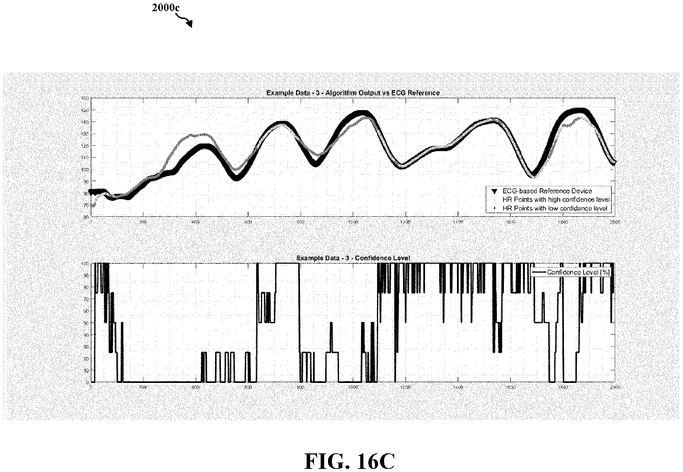

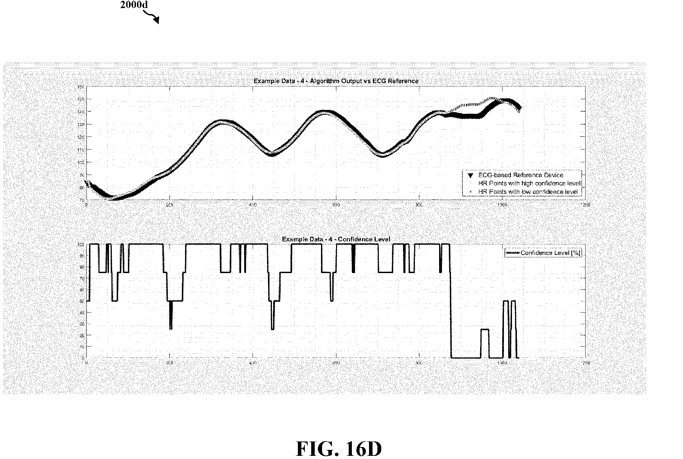

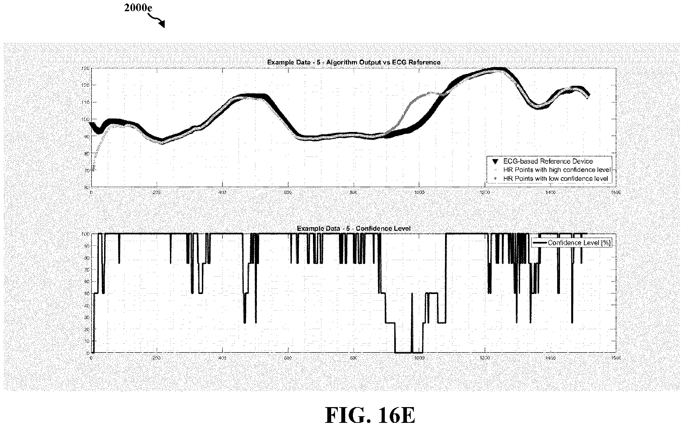

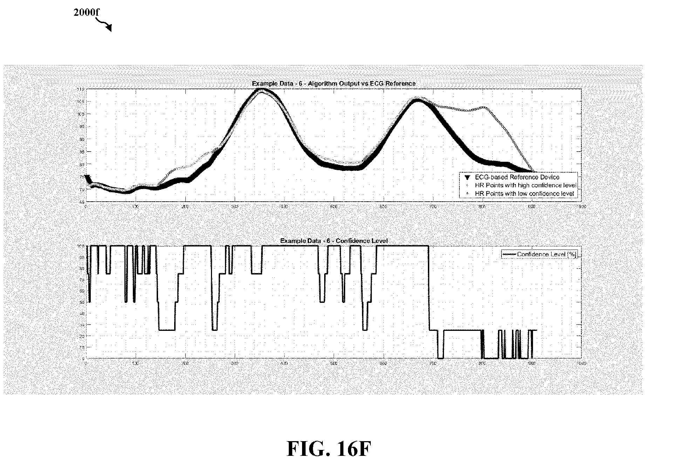

FIGs. 16A-16F are diagrams 2000a-2000f, respectively, illustrating good examples of confidence level analysis. In each of FIGs. 20A-20M, the upper diagram portion includes a solid line representing a polar reference, while heart rate points with a high and low confidence levels via algorithm outputs are shown as true and false based on their difference from the polar reference. The lower diagram portion includes confidence intervals between the reference and algorithm output.

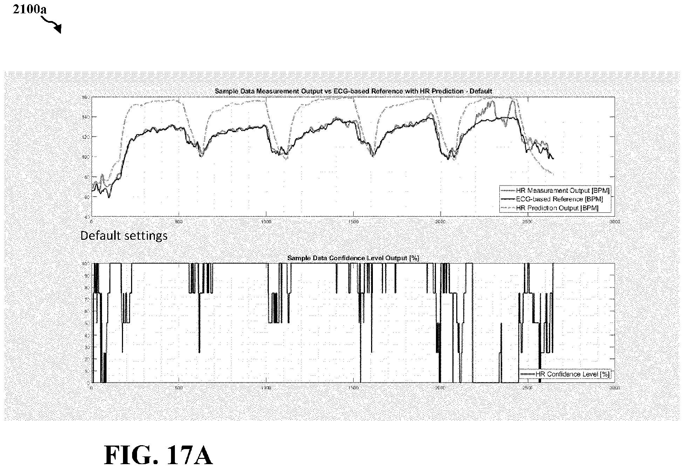

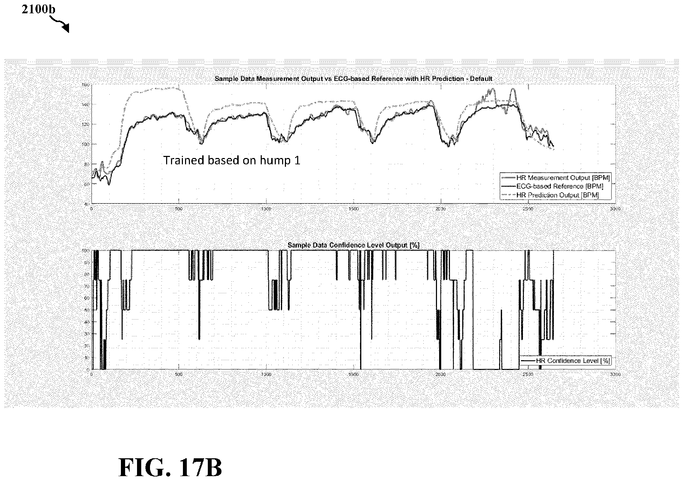

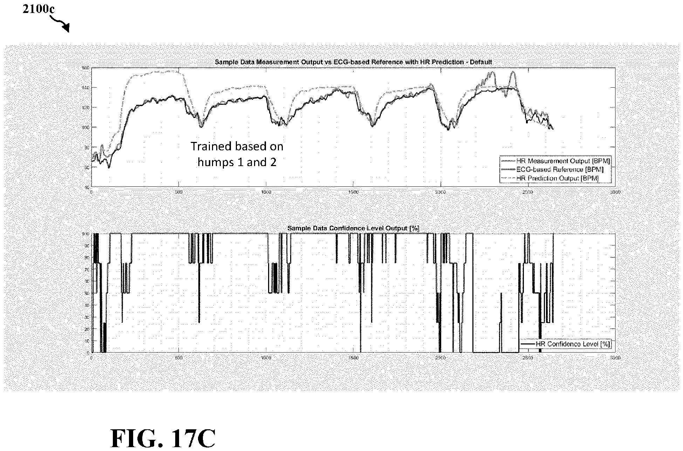

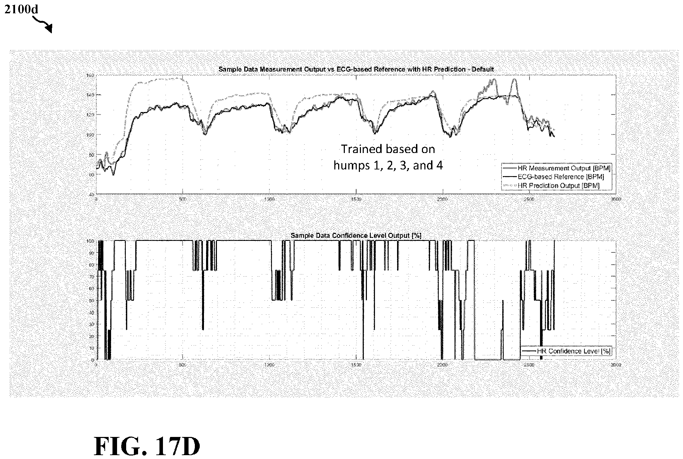

FIGs. 17A-17D are diagrams 2100a-2100d, respectively, illustrating dynamic prediction model development for a user engaged in a running activity. As shown in FIG. 17A, default settings can show an algorithm output, a polar reference, and prior measured heart rates, with accuracy shown according to confidence levels. As shown in FIG. 17B, training based on a first hump can show an algorithm output, a polar reference, and prior measured heart rates, with accuracy shown according to confidence levels. As shown in FIG. 17C, training based on a first and a second hump can show an algorithm output, a polar reference, and prior measured heart rates, with accuracy shown according to confidence levels. As shown in FIG. 17D, adjustment based on a first, second, third, and fourth hump can show an algorithm output, a polar reference, and prior measured heart rates, with accuracy shown according to confidence levels.

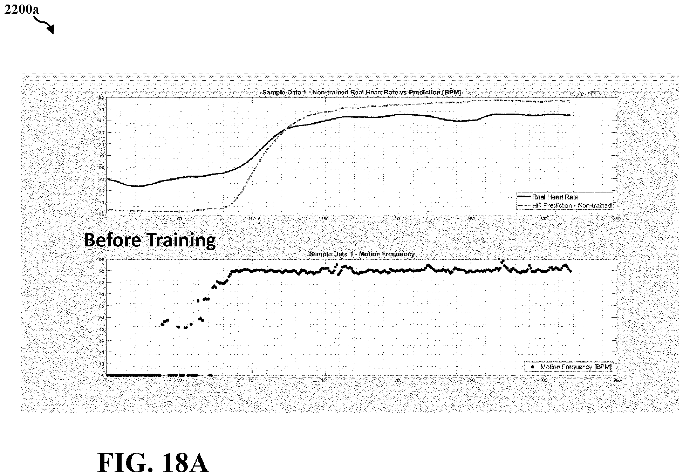

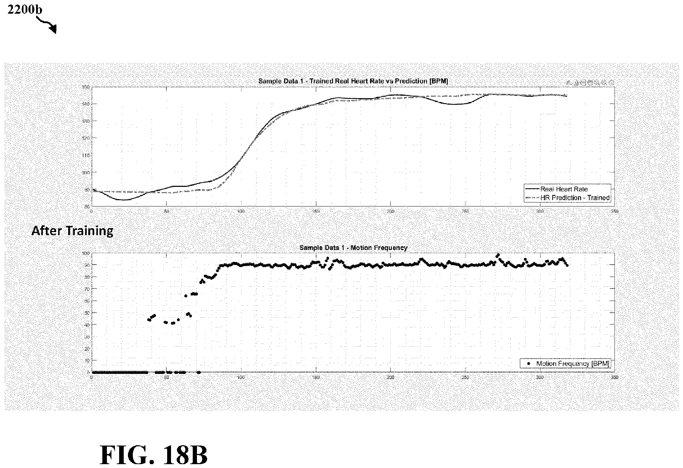

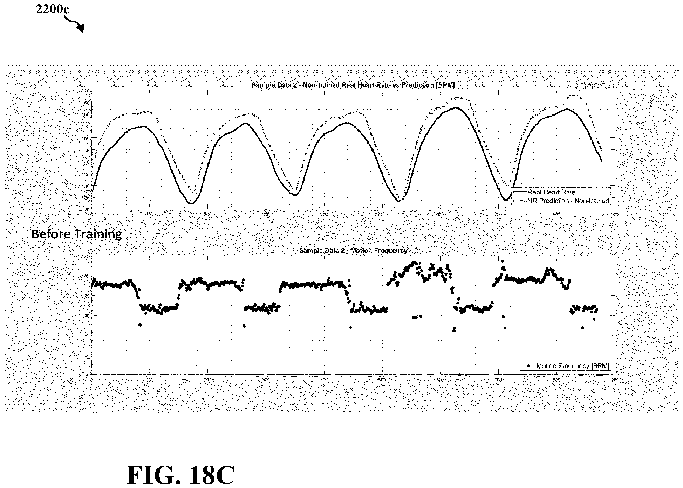

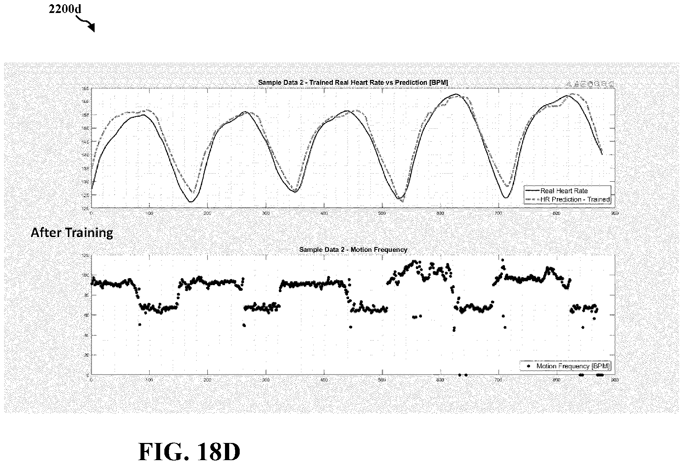

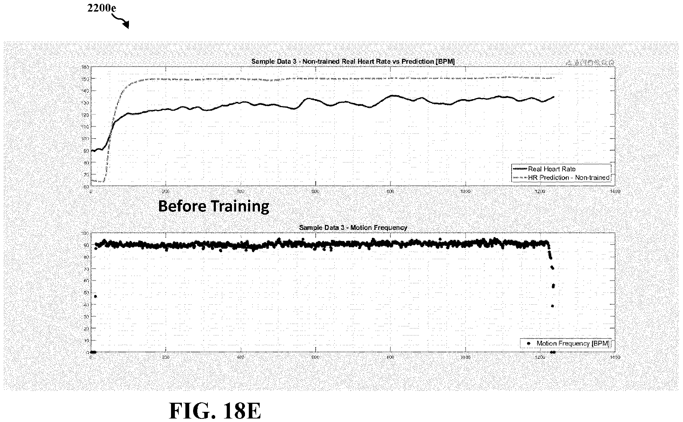

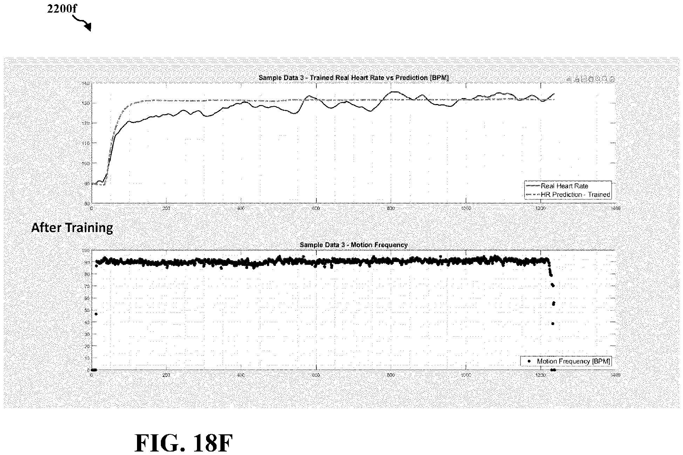

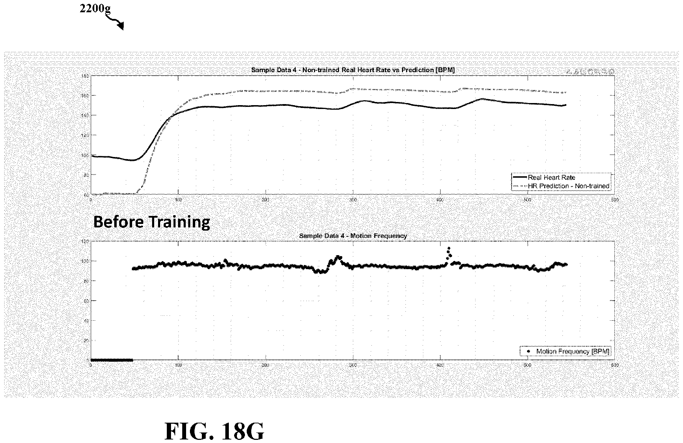

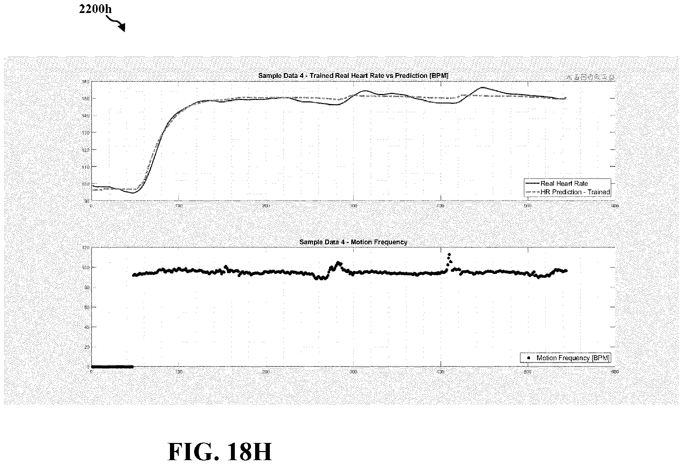

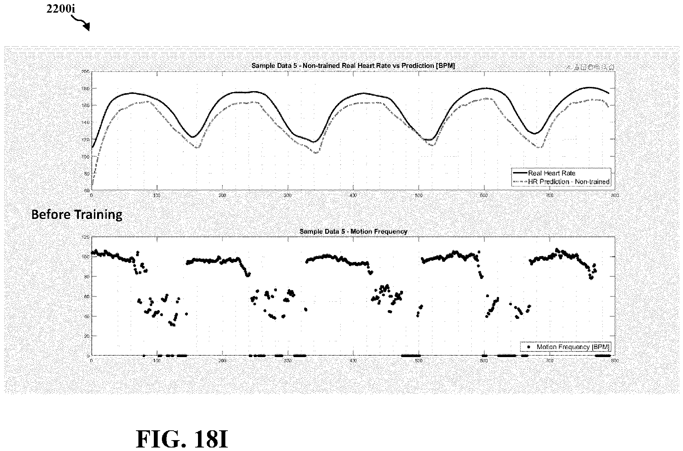

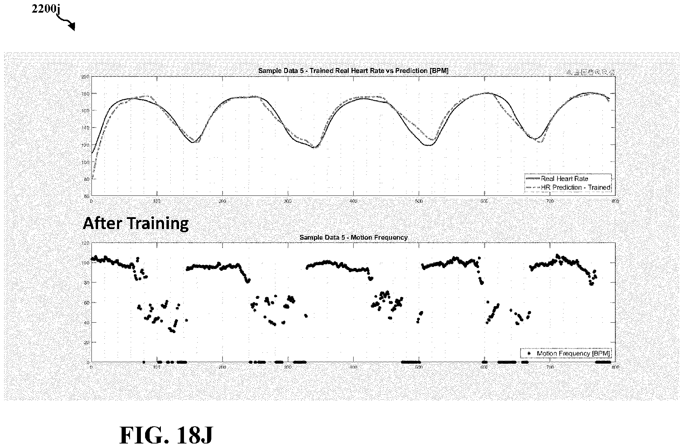

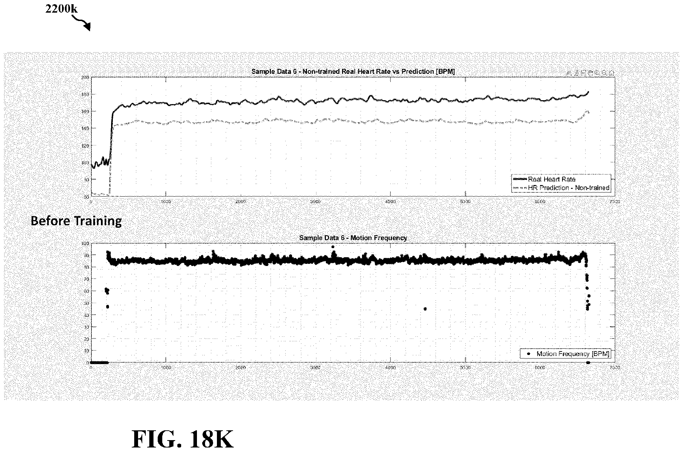

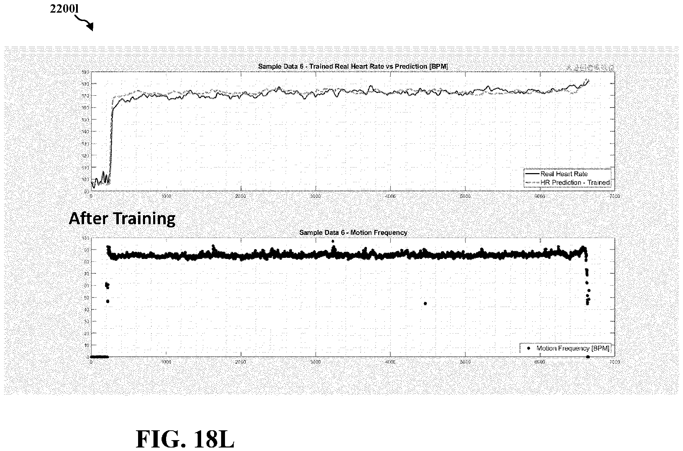

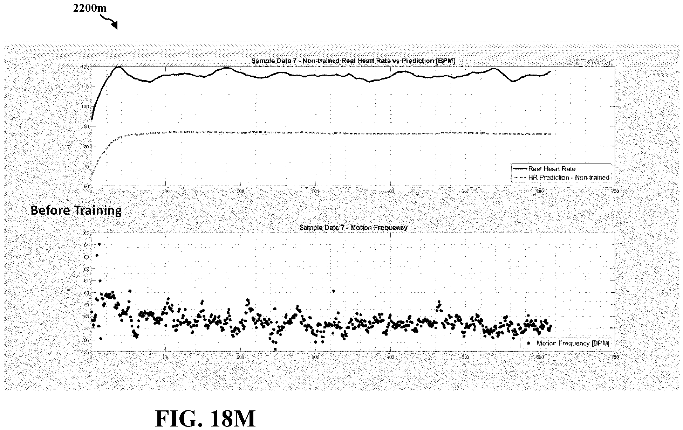

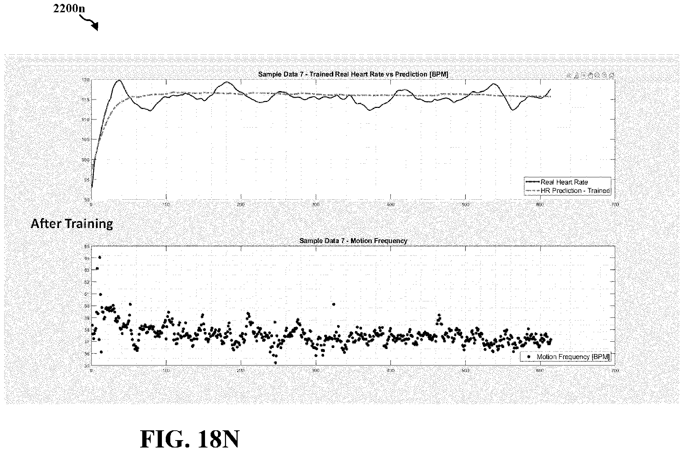

FIGs. 18A-18N are diagrams 2200a-2200n, respectively, illustrating before training diagrams for non-trained real heart rate versus prediction reference and motion frequency (in BPM) and after training diagrams for trained.

It is understood that the specific order or hierarchy of blocks in the processes / flowcharts disclosed is an illustration of exemplary approaches. Based upon design preferences, it is understood that the specific order or hierarchy of blocks in the processes / flowcharts may be rearranged. Further, some blocks may be combined or omitted. The accompanying method claims present elements of the various blocks in a sample order and are not meant to be limited to the specific order or hierarchy presented.

The previous description is provided to enable any person skilled in the art to practice the various aspects described herein. Various modifications to these aspects will be readily apparent to those skilled in the art, and the generic principles defined herein may be applied to other aspects Thus, the claims are not intended to be limited to the aspects shown herein but is to be accorded the full scope consistent with the language claims, wherein reference to an element in the singular is not intended to mean "one and only one" unless specifically so stated, but rather "one or more." The word "exemplary" is used herein to mean "serving as an example, instance, or illustration." Any aspect described herein as "exemplary" is not necessarily to be construed as preferred or advantageous over other aspects. Unless specifically stated otherwise, the term "some" refers to one or more. Combinations such as "at least one of A, B, or C," "one or more of A, B, or C," "at least one of A, B, and C," "one or more of A, B, and C," and "A, B, C, or any combination thereof" include any combination of A, B, and/or C, and may include multiples of A, multiples of B, or multiples of C. Specifically, combinations such as "at least one of A, B, or C," "one or more of A, B, or C," "at least one of A, B, and C," "one or more of A, B, and C," and "A, B, C, or any combination thereof" may be A only, B only, C only, A and B, A and C, B and C, or A and B and C, where any such combinations may contain one or more member or members of A, B, or C. All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. The words "module," "mechanism," "element," "device," and the like may not be a substitute for the word "means." As such, no claim element is to be construed as a means plus function unless the element is expressly recited using the phrase "means for."

There follows a list of numbered features representing particular embodiments of the present disclosure. Where a numbered feature refers to one or more earlier numbered features then those features should be considered together in combination.
1. A device for determining a personalized heart rate, comprising:
   one or more heart rate sensors configured to obtain heart rate values for a user for a time period;
   one or more movement sensors configured to obtain movement values for the user for the time period, wherein the movement values are associated with an activity type;
   wherein one or more of the heart rate values are associated with a signal quality that is less than a signal quality threshold;
   one or more processors configured to execute a personalized heart rate prediction model, trained on user-specific movement data and user-specific heart rate data associated with signal qualities greater than the signal quality threshold, to:
      receive the heart rate values and the activity type or the movement values; and
      generate, based on the heart rate values and the activity type or the movement values, a user-specific predicted heart rate for at least a part of the time period; and
      a user interface configured to output the user-specific predicted heart rate as the user heart rate for at least the part of the time period.
2. The device of feature 1, wherein the activity type comprises a non-resting activity or other activity type.
3. The device of feature 2, wherein the non-resting activity type is running, walking, rowing, or biking.
4. The device of any of the preceding features, wherein the one or more heart rate sensors are configured to obtain a heart rate signal having a plurality of different frequency components, wherein the one or more processors are configured to execute the personalized heart rate prediction model to select one of the plurality of different frequency components as corresponding to the heart rate value.
5. The device of any of the preceding features, wherein the personalized heart rate prediction model is trained on user-specific movement data and user-specific heart rate data associated with signal qualities greater than the signal quality threshold by storing the user-specific movement data and user-specific heart rate data in a lookup table associated with the activity type.
6. The device of feature 5, wherein the lookup table is generated based on a plurality of user-specific movement data and user-specific heart rate data for the activity type.
7. The device of feature 6, wherein the activity type is determined based on motion frequency and density.
8. The device of any of the preceding features, wherein the one or more movement sensors comprise a 3-axis accelerometer and a gyroscope.
9. The device of any of the preceding features, wherein the one or more heart rate sensors comprise a photoplethysmography (PPG) sensor.
10. The device of feature 5, wherein, to generate the user-specific predicted heart rate for at least a part of the time period, the one or more processors are further configured to:
   evaluate signal quality with a confidence interval for the measured heart rate for a period of time.
11. The device of feature 10, wherein, to generate the user-specific predicted heart rate for at least a part of the time period, when the evaluated signal quality has a low confidence interval, the one or more processors are further configured to:
   calculate a maximum prediction heart rate for the activity; and
   perform post-processing smoothing prediction for the heart rate.
12. A method for determining a personalized heart rate for a user of a heart rate prediction apparatus, the method comprising one or more processors of the prediction apparatus:
   obtaining heart rate values for the user for a time period via one or more heart rate sensors;
   obtaining movement values for the user for the time period via one or more movement sensors, wherein the movement values are associated with an activity type;
   wherein one or more of the heart rate values are associated with a signal quality that is less than a signal quality threshold;
   executing, via one or more processors, a personalized heart rate prediction model, trained on user-specific movement data and user-specific heart rate data associated with signal qualities greater than the signal quality threshold, wherein the personalized heart rate prediction model:
      receives the heart rate values and the activity type or the movement values; and
      generates, based on the heart rate values and the activity type or the movement values,
      a user-specific predicted heart rate for at least a part of the time period; and
      outputting the user-specific predicted heart rate as the user heart rate for at least the part of the time period via a user interface.
13. The method of feature 12, wherein the activity type comprises a non-resting activity type.
14. The method of feature 13, wherein the non-resting activity type is running, walking, rowing, biking, or other activity.
15. The method of any of features 12 to 14, wherein obtaining heart rate values further comprises obtaining a heart rate signal having a plurality of frequency components, and wherein executing the personalized heart rate prediction model further comprises selecting one of the plurality of different frequency components as corresponding to the heart rate value.
16. The method of any of features 12 to 15, wherein the personalized heart rate prediction model is trained on user-specific movement data and user-specific heart rate data associated with signal qualities greater than the signal quality threshold by storing the user-specific movement data and user-specific heart rate data in a lookup table associated with the activity type for use by a linear function, non-linear function, machine-learning model, or other model.
17. The method of feature 14, wherein the lookup table is generated based on a plurality of user-specific movement data and user-specific heart rate data for the activity type.
18. The method of feature 17, wherein the activity type is determined based on motion frequency and density.
19. The method of any of features 12 to 14, wherein the one or more heart rate sensors comprise a photoplethysmography (PPG) sensor.
20. A system for providing a personalized heart rate for a user of a heart rate monitoring apparatus, the system comprising:
   one or more heart rate sensors;
   one or more movement sensors; and
   the heart rate monitoring apparatus, comprising:
      one or more processors;
      one or more memory; and
      a display,
   wherein the one or more processors of the heart rate monitoring apparatus are configured to:
   measure a heart rate of the user during an activity for a period of time via the one or more heart rate sensors;
   measure movement values for the user during the activity for the period of time via the one or more movement sensors;
      determine an activity classification based on the movement values;
   evaluate the signal quality of the measured heart rate for the period of time, wherein if a confidence level of the measured heart rate is low, finding a predicted heart rate for the user during the period of time based on the movement values in a personalized lookup table associated with the activity; and
   display a heart rate diagram based on the heart rate of the user during the activity via a device display.

## Claims

1. A device for determining a personalized heart rate, comprising:
one or more heart rate sensors configured to obtain heart rate values for a user for a time period;
one or more movement sensors configured to obtain movement values for the user for the time period, wherein the movement values are associated with an activity type;
wherein one or more of the heart rate values are associated with a signal quality that is less than a signal quality threshold;
one or more processors configured to execute a personalized heart rate prediction model, trained on user-specific movement data and user-specific heart rate data associated with signal qualities greater than the signal quality threshold, to:
receive the heart rate values and the activity type or the movement values; and
generate, based on the heart rate values and the activity type or the movement values, a user-specific predicted heart rate for at least a part of the time period; and
a user interface configured to output the user-specific predicted heart rate as the user heart rate for at least the part of the time period.

2. The device of claim 1, wherein the activity type comprises a non-resting activity or other activity type; wherein optionally the non-resting activity type is any one or more of: running, walking, rowing, or biking.

3. The device of claim any of the preceding claims, wherein the one or more heart rate sensors are configured to obtain a heart rate signal having a plurality of different frequency components, wherein the one or more processors are configured to execute the personalized heart rate prediction model to select one of the plurality of different frequency components as corresponding to the heart rate value.

4. The device of any of the preceding claims, wherein the personalized heart rate prediction model is trained on user-specific movement data and user-specific heart rate data associated with signal qualities greater than the signal quality threshold by storing the user-specific movement data and user-specific heart rate data in a lookup table associated with the activity type.

5. The device of claim 4, wherein the lookup table is generated based on a plurality of user-specific movement data and user-specific heart rate data for the activity type; wherein optionally the activity type is determined based on motion frequency and density.

6. The device of any of the preceding claims, wherein:
a) the one or more movement sensors comprise a 3-axis accelerometer and a gyroscope; and/or
b) the one or more heart rate sensors comprise a photoplethysmography (PPG) sensor.

7. The device of claim 4, wherein, to generate the user-specific predicted heart rate for at least a part of the time period, the one or more processors are further configured to:
evaluate signal quality with a confidence interval for the measured heart rate for a period of time.

8. The device of claim 7, wherein, to generate the user-specific predicted heart rate for at least a part of the time period, when the evaluated signal quality has a low confidence interval, the one or more processors are further configured to:
calculate a maximum prediction heart rate for the activity; and
perform post-processing smoothing prediction for the heart rate.

9. A method for determining a personalized heart rate for a user of a heart rate prediction apparatus, the method comprising one or more processors of the prediction apparatus:
obtaining heart rate values for the user for a time period via one or more heart rate sensors;
obtaining movement values for the user for the time period via one or more movement sensors, wherein the movement values are associated with an activity type;
wherein one or more of the heart rate values are associated with a signal quality that is less than a signal quality threshold;
executing, via one or more processors, a personalized heart rate prediction model, trained on user-specific movement data and user-specific heart rate data associated with signal qualities greater than the signal quality threshold, wherein the personalized heart rate prediction model:
receives the heart rate values and the activity type or the movement values; and
generates, based on the heart rate values and the activity type or the movement values, a user-specific predicted heart rate for at least a part of the time period; and
outputting the user-specific predicted heart rate as the user heart rate for at least the part of the time period via a user interface.

10. The method of claim 9, wherein the activity type comprises a non-resting activity type; wherein optionally the non-resting activity type is any one or more of: running, walking, rowing, biking, or other activity.

11. The method of claims 9 or 10, wherein obtaining heart rate values further comprises obtaining a heart rate signal having a plurality of frequency components, and wherein executing the personalized heart rate prediction model further comprises selecting one of the plurality of different frequency components as corresponding to the heart rate value.

12. The method of any of claims 9 to 11, wherein the personalized heart rate prediction model is trained on user-specific movement data and user-specific heart rate data associated with signal qualities greater than the signal quality threshold by storing the user-specific movement data and user-specific heart rate data in a lookup table associated with the activity type for use by a linear function, non-linear function, machine-learning model, or other model.

13. The method of claim 12, wherein the lookup table is generated based on a plurality of user-specific movement data and user-specific heart rate data for the activity type; wherein optionally the activity type is determined based on motion frequency and density.

14. The method of any of claims 9 to 13, wherein the one or more heart rate sensors comprise a photoplethysmography (PPG) sensor.

15. A system for providing a personalized heart rate for a user of a heart rate monitoring apparatus, the system comprising:
one or more heart rate sensors;
one or more movement sensors; and
the heart rate monitoring apparatus, comprising:
one or more processors;
one or more memory; and
a display,
wherein the one or more processors of the heart rate monitoring apparatus are configured to:
measure a heart rate of the user during an activity for a period of time via the one or more heart rate sensors;
measure movement values for the user during the activity for the period of time via the one or more movement sensors;
determine an activity classification based on the movement values;
evaluate the signal quality of the measured heart rate for the period of time, wherein if a confidence level of the measured heart rate is low, finding a predicted heart rate for the user during the period of time based on the movement values in a personalized lookup table associated with the activity; and
display a heart rate diagram based on the heart rate of the user during the activity via a device display.
